# EUROPÄISCHE PATENTANMELDUNG

(11) **EP 0 864 307 A1**
(43) Veröffentlichungstag der Anmeldung: **16.09.1998**
(21) Anmeldenummer: 98103504.1
(22) Anmeldetag: 27.02.1998
(51) Int. Cl.: A61F 2/80, A61F 2/50

(54) **Schlauchförmiges Fasermaterial und Verfahren zur Herstellung eines Prothesenschaftes aus einem solchen Material**

(30) Priorität: 12.03.1997 DE 19710230
(71) Anmelder: BIEDERMANN MOTECH GmbH, 78054 Villingen-Schwenningen (DE)
(72) Erfinder: Biedermann, Lutz, 78048 VS-Villingen (DE); Piro, Markus, 78078 Niedereschach (DE)
(74) Vertreter: Prüfer, Lutz H., Dipl.-Phys.

(57) **Zusammenfassung**

Es wird ein schlauchförmiges Fasermaterial zum Bilden eines zur Aufnahme eines Stumpfes an seinem einen Ende offenen Prothesenschaftes (10) vorgegebener Länge angegeben, dessen anderes Ende zum Halten eines Verbindungselementes (5) geschlossen ist und zum Hindurchführen eines Teiles (7) des Verbindungselementes (5) nach außen eine dem Teil (7) entsprechende Öffnung aufweist. Der Schlauch wird als Ausgangsmaterial länger gewählt als die Länge des zu bildenden Prothesenschaftes (10). Der Schlauch wird mit Hilfe eines Ringes (2) in einer vorgegebenen Länge von außen ringförmig zusammengefaßt, so daß ein von der Öffnung (1) her gesehen erster Abschnitt (3) gebildet wird. Der übrige zweite Abschnitt (4) wird dann über die ringförmige Fassung auf den ersten Abschnitt (3) hin umgeschlagen. Zum Bilden des Prothesenschaftes (10) wird das Material mit einem thermoplastischen Harz getränkt.

## Beschreibung

Die Erfindung betrifft ein schlauchförmiges Fasermaterial zum Bilden eines zur Aufnahme eines Stumpfes an seinem einen Ende offenen Prothesenschaftes und ein Verfahren zur Herstellung eines solchen Prothesenschaftes aus einem derartigen schlauchförmigen Material.

Aus der US-PS 5,228,164 ist ein Materialsatz zum Bilden eines Negativ-Abdruckes eines Körperteiles bekannt.

Aus der US-PS 5,507,834 ist ein Silikonstrumpf bekannt, der an seinem dem offenen Ende gegenüberliegenden geschlossenen Ende ein Verbindungselement aufweist, in welches ein Bolzen einschraubbar ist, der mit einem mechanischen Fußpaßteil verbindbar ist.

Aus der WO 96/29033 und aus der WO 95/05792 sind jeweils eine Fassung und ein innerer Strumpf zur Aufnahme eines Stumpfes bekannt.

Aufgabe der Erfindung ist es, ein schlauchförmiges Fasermaterial zu schaffen, mit dem auf einfache Weise ein mit einem mechanischen Gliedpaßteil zu verbindender Prothesenschaft ohne die Notwendigkeit des Herstellens einer Zwischenform möglich wird. Ferner soll ein Verfahren zur Herstellung eines derartigen Prothesenschaftes aus einem solchen schlauchförmigen Material angegeben werden.

Diese Aufgabe wird durch das in Patentanspruch 1 gekennzeichnete schlauchförmige Fasermaterial bzw. durch das in Patentanspruch 5 gekennzeichnete Verfahren zur Herstellung eines Prothesenschaftes aus einem solchen schlauchförmigen Material gelöst.

Weiterbildungen der Erfindung sind in den Unteransprüchen gekennzeichnet.

Weitere Merkmale und Zweckmäßigkeiten der Erfindung ergeben sich aus der Beschreibung eines Ausführungsbeispieles anhand der Figuren. Von den Figuren zeigen:

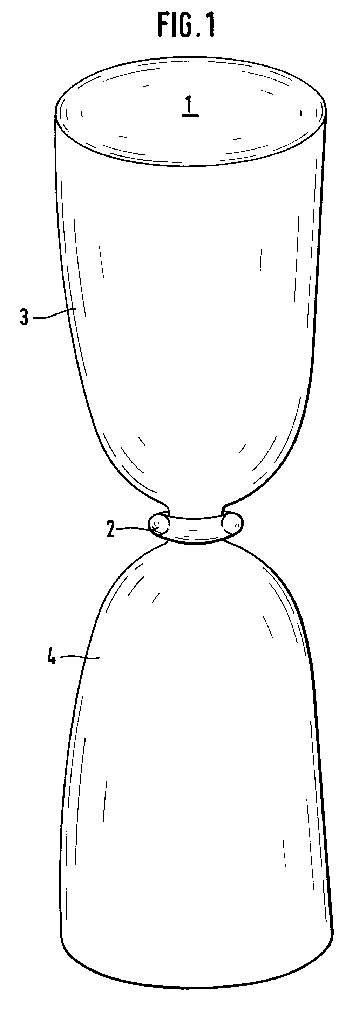

- Fig. 1: eine Seitenansicht des schlauchförmigen Fasermaterials mit der ringförmigen Einfassung;
- Fig. 2: eine Schnittdarstellung des Prothesenschaftes mit eingesetztem Verbindungselement; und

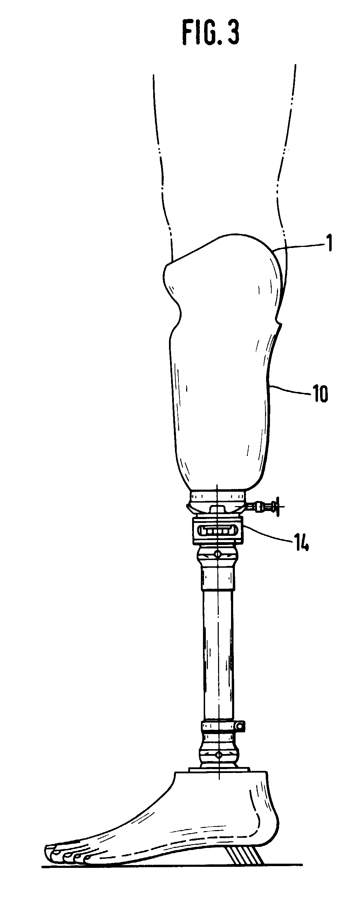

- Fig. 3: eine Seitenansicht einer Prothese mit aufgenommenem Schaft.

Das Ausgangsmaterial ist ein schlauchförmiges Fasermaterial, welches aus Glasfasern gestrickt oder gewirkt ist. Für die Bildung eines Prothesenschaftes 10 wird ein Stück eines solchen Schlauchmateriales abgetrennt, welches im wesentlichen gleich der zweifachen Länge des zu bildenden Prothesenschaftes 10 ist. In einem Abstand von dem einen offenen Ende 1 wird der Schlauch mit einem Ring 2 umfaßt und quasi zusammengeschnürt.

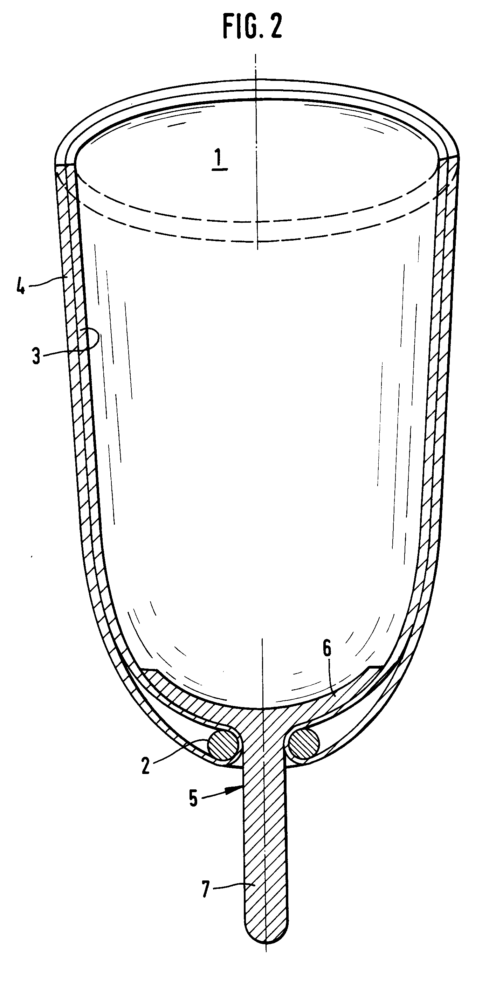

Der Abstand des Ringes 2 von dem offenen Ende 1 wird so gewählt, daß ein so gebildeter erster Abschnitt 3 gleich der Länge des zu bildenden Prothesenschaftes 10 ist. Ein dem ersten Abschnitt 3 gegenüberliegender zweiter Abschnitt 4 wird dann über den Ring 2 und den ersten Abschnitt 3 zurückgeschlagen, so daß das in Fig. 2 gezeigte doppelwandige Teil 11 mit einem eine zentrale Öffnung 12 aufweisenden Boden 13 entsteht.

Anschließend wird in der in Fig. 2 gezeigten Weise ein Verbindungselement 5 in den Boden 13 eingesetzt, welches einen tellerförmigen Abschnitt 6 und einen damit einstückig ausgebildeten bolzenförmigen Abschnitt 7 aufweist. Das Einsetzen erfolgt in der in Fig. 2 gezeigten Weise so, daß der bolzenförmige Abschnitt 6 durch die Öffnung 12 geführt wird und dann der Abschnitt 6 innen am Boden 13 anliegt. Der Ring 2 ist in seinem Durchmesser so bemessen, daß der bolzenförmige Abschnitt 7 gerade durch die Öffnung 12 hindurch paßt. Die von dem Ring 2 durch Zusammenfassen des Schlauches gebildete Öffnung 12 ist also in ihrem Durchmesser im wesentlichen gleich dem Durchmesser des bolzenförmigen Abschnittes 7. Der bolzenförmige Abschnitt 7 dient in bekannter Weise zum Verbinden des Prothesenschaftes 10 mit einem mechanischen Arm- oder Beinpaßteil 14. Der tellerförmige Abschnitt 6 ist so ausgebildet, daß er auf dem Grund des dem offenen Ende 1 gegenüberliegenden Boden 13 glatt anliegt und den bolzenförmigen Abschnitt 7 trägt.

Nachdem das schlauchförmige Material und das Verbindungselement derart vorbereitet sind, wird das Fasermaterial mit einem Harz getränkt. Als Harz wird ein Material mit selbsthärtenden Eigenschaften gewählt, welches vorzugsweise mit Wasser aktivierbar ist und bei geringer Wärmeabgabe aushärtet. Ein vorzugsweises Beispiel für ein solches Harz ist Polyurethanharz. Das Harz soll im ausgehärteten Zustand vorzugsweise thermoplastische Eigenschaften aufweisen.

Das so vorgefertigte Fasermaterial wird zum Bilden eines Prothesenschaftes 10 in das aktivierende Wasser eingetaucht und dann über einen gegebenenfalls zuvor mit einem dünnen Strumpf überzogenen Stumpf gezogen und so angeformt, daß der gewünschte Sitz erreicht wird. Dazu wird das doppelwandige Teil 11 an den Stumpf angedrückt und nach dem Aushärten am oberen freien Rand zugeschnitten. Gegebenenfalls kann der Rand vor dem Aushärten auch in einer Weise umgebörtelt werden, die ein komfortables Tragen ermöglicht.

Stellen sich beim Tragen des Prothesenschaftes 10 Druckstellen heraus, werden diese durch Erwärmen des Materials an den relevanten Stellen nachgeformt.

In dem oben beschriebenen Ausführungsbeispiel ist das Fasermaterial aus Glasfasern gebildet. In einer abgewandelten Ausführungsform wird das Fasermaterial aus einer Kombination von Glasfasern und anderen Verstärkungsfasern wie Karbon- oder Kevlarfasern gebildet.

## Patentansprüche

1. Schlauchförmiges Fasermaterial zum Bilden eines zur Aufnahme eines Stumpfes an seinem einen Ende (1) offenen Prothesenschaftes (10) vorgegebener Länge, dessen anderes Ende zum Halten eines Verbindungselementes (5) geschlossen ist und zum Hindurchführen eines Teiles (7) des Verbindungselementes (5) nach außen eine dem Teil (7) entsprechende Öffnung (12) aufweist, und bei dem der Schlauch länger als die Länge des zu bildenden Prothesenschaftes (10) gewählt und ein erster Abschnitt (3) in der vorgegebenen Länge von außen ringförmig zusammengefaßt und der einen zweiten Abschnitt (4) bildende übrige Teil über die ringförmige Fassung auf dem ersten Abschnitt (3) zurückgeschlagen ist.

2. Schlauchförmiges Fasermaterial nach Anspruch 1, dadurch gekennzeichnet, daß die Länge des zweiten Abschnitts (4) im wesentlichen gleich dem ersten Abschnitt (3) ist.

3. Schlauchförmiges Fasermaterial nach Anspruch 1 oder 2, dadurch gekennzeichnet, daß das Fasermaterial aus Glasfasern gebildet ist.

4. Schlauchförmiges Fasermaterial nach Anspruch 1 oder 2, dadurch gekennzeichnet, daß das Material aus einem Gemisch von Glasfasern und anderen Verstärkerfasern wie Karbon- oder Kevlarfasern gebildet ist.

5. Schlauchförmiges Fasermaterial nach einem der Ansprüche 1 bis 4, dadurch gekennzeichnet, daß der Schlauch gestrickt oder gewirkt ist.

6. Schlauchförmiges Fasermaterial nach einem der Ansprüche 1 bis 5, dadurch gekennzeichnet, daß das Material mit einem selbsthärtenden Harz getränkt ist.

7. Schlauchförmiges Fasermaterial nach Anspruch 6, dadurch gekennzeichnet, daß das Harz wasseraktivierbar ist.

8. Schlauchförmiges Fasermaterial nach Anspruch 6 oder 7, dadurch gekennzeichnet, daß das Harz nach dem Aushärten thermoplastisch ist.

9. Schlauchförmiges Fasermaterial nach einem der Ansprüche 1 bis 8, dadurch gekennzeichnet, daß durch die von dem Ring (2) gebildete Öffnung (12) im Boden (13) ein nach außen hervorstehender bolzenförmiger Abschnitt (7) des Verbindungselementes (5) geführt ist.

10. Verfahren zur Herstellung eines Prothesenschaftes aus einem schlauchförmigen Material nach einem der Ansprüche 1 bis 9, dadurch gekennzeichnet, daß das mit einem Harz getränkte Fasermaterial über den aufzunehmenden Stumpf gebracht und durch Andrücken von außen an den Stumpf angeformt wird.

11. Verfahren zur Herstellung eines Prothesenschaftes aus einem schlauchförmigen Material nach Anspruch 10, dadurch gekennzeichnet, daß das Harz mit Wasser aktiviert wird.
